# EUROPEAN PATENT APPLICATION

(11) **EP 1 336 604 A1**
(43) Date of publication of application: **20.08.2003**
(21) Application number: 02003417.9
(22) Date of filing: 14.02.2002
(51) Int. Cl.: C07D 209/48, C07D 291/04, A61K 31/40, A61K 31/41

(54) **Sulfoximine derivatives as MMP-inhibitors or antibiotics**

(71) Applicant: Wettstein, Philipp, Dr., 4410 Liestal (CH); Müller, Jürgen F.K., Dr., 79112 Freiburg-Tiengen (DE)
(72) Inventor: Wettstein, Philipp, Dr., 4410 Liestal (CH); Müller, Jürgen F.K., Dr., 79112 Freiburg-Tiengen (DE)
(74) Representative: Maucher, Wolfgang, Dipl.-Ing.

(57) **Abstract**

The invention relates to the use of a sulfonamidimide of the formula I, 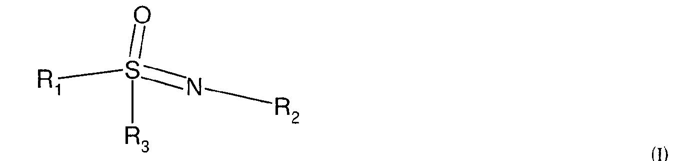 wherein R₁, R₂ and R₃ have the meanings given in the specification;
or a tautomer thereof; or a salt of said compound or tautomer where salt-forming groups are present;
for the manufacture of a pharmaceutical preparation useful in the treatment of a matrix metalloprotease associated disease or as antibiotics, as well as corresponding methods of treatment. Also novel compounds of the formula I are disclosed.

## Description

### Summary of the Invention

The invention relates to the use of an organic sulfonamidamide in the treatment of a matrix metalloprotease (MMP) dependent disease and/or its use as antibiotic; the use of an organic sulfonamidamide in the preparation of a pharmaceutical composition for the treatment of a MMP dependent disease and/or for antibiotic treatment; a pharmaceutical preparation comprising an organic sulfonamidamide; a method of treatment comprising administering said compound to an animal, especially a human, suffering from a MMP dependent disease and/or a disease accessible to antibiotic treatment, as well as to novel sulfonamidamides, processes for their manufacture and their use as pharmaceuticals.

### Background of the invention

A lot of research in the last decades did focus on the development of modifications of peptides leading to peptide analogs, as the usefulness of peptides for medical purposes is usually seriously hampered in view of their low bioavailability and rapid degradation. Dealing inter alia with attempts to find analogues for renin inhibitors and HIV protease inhibitors, but also matrix metalloproteinase inhibitors, hormone analogs and various other, an incredible amount of possible peptide mimetics has been created. The major goal is to introduce such peptide mimetics without hampering or while even improving the biological activity of the original peptides, at the same time diminishing the mentioned disadvantages. Among the modifications, the following can be mentioned: Deletion, addition or replacement of amino acids; cyclizations; replacement of the peptide backbone by, for example isosteric, surrogates; replacement of the backbone by totally new scaffolds; or the addition of groups, e.g. by glycosylation, in order to modify, for example, the solubility and/or pharmacokinetic behaviour of the peptides.

Matrix metalloproteases or matrix metalloproteinases (MMPs) are a family of Zn²⁺-dependent endopeptidases that play an important role in the extracellular matrix turnover and are associated with tissue destruction in various diseases such as rheumatoid arthritis. Among the members of the MMP family are gelatinase A (MMP-2) and gelatinase B (MMP-9). For example, proMMP-2 is secreted by human rheumatoid synovial fibroblasts, to a minor extent also proMMP-9 (se, for example, e H. Smolian et al., Biol. Chem. 382, 1491-9 (2001), and references cited therein), the inactive proenzymes. Another Matrix metalloproteinase is MMP-7 (also called Matrilysin or Uterine Metalloproteinase) which, inter alia, was detected in neoplastic epithelial tumors. Most of the MMPs are activated by a variety of proteinases, e.g. stromelysin (itself an MMP, also called MMP-3), cathepsin G, and plasma membrane anchored, so-called membrane type matrix metalloproteinases. Once activated, they hydrolyse type IV collagen, the major structural component of the basal membrane. The basal membrane is a barrier that must be overcome in processes such as leukodiapedesis, tumor invasion and the metastasis of tumor cells.

Therefore, inhibition of the activity of the MMPs is a means allowing for the treatment of tumor diseases, especially lung carcinoma, the suppression of tumor metastase formation, rheumatoid arthritis, and even multiple sclerosis. For the treatment of these diseases, an ever-existing strong-felt need exists for novel and effective pharmaceuticals.

It is thus a problem to be solved by the present invention to provide for novel matrix metalloproteinase inhibitors (MMP inhibitors) that allow for the treatment of these types of diseases.

Another problem to be solved relates to the problems associated with the increasing number of infectious strains of pathogenic bacteria, mycoplasms, fungi or virus and other pathogenic microorganisms, like protozoa, that show resistance against many of the customary antibiotics used hitherto in the treatment of infectious diseases.

The result of this development of resistance is that many infectious diseases that were regarded as more or less defeated now pose a serious threat for the patients. Thus, for example, resistant versions of tuberculosis are emerging in many countries in eastern Europe and Asia, endangering the former successes in the combat of this disease with appropriate standard antibiotics, as are pathogens for diseases in infectious hospitalism, like Staphylococcus aureus or other pyogenic microorganisms.

This leads to a continuous requirement to find and use novel antibiotic compounds.

### General Description of the Invention

It has now surprisingly been found that peptide mimetics of the sulfonamidimide type are both inhibitors of proteinases, especially matrix metalloproteinase inhibitors, thus enabling the treatment of MMP-associated diseases, and display antibiotic activity, thus providing a novel class of antibiotically active compounds and adding to the arsenal of compounds useful in combating infectious diseases.

Therefore, the invention relates to the use of or methods of using this compound class as proteinase inhibitors, specifically as MMP inhibitors, and as antibiotics.

The invention also relates to novel compounds belonging to the sulfonamidimide class of compounds, as well as their use or methods comprising their administration to an animal, especially a human.

The novel use and the novel compounds offer an advantageous way to treat many diseases, especially those mentioned above and below.

### Detailed Description of the Invention

The invention relates especially to the use of a compound of the formula I, 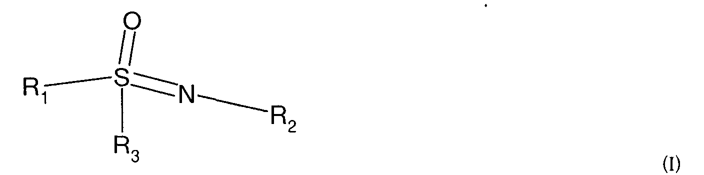 wherein R₁ is an unsubstituted or substituted moiety selected from the group consisting of alkyl, cycloalkyl, aryl and heteroaryl;
R₂ is an unsubstituted or substituted moiety selected from alkyl, cycloalkyl, aryl and heteroaryl, or is amino, halogen, substituted amino, aminoacyl or substituted hydroxy; and
R₃ is an unsubstituted or substituted moiety selected from alkyl, alkenyl, alkinyl, cycloalkyl, aryl and heteroaryl, or is substituted amino, substituted mercapto or substituted hydroxy,
or R₂ and R₃ together form a bridge in the form of a bivalent organic moiety;
or a tautomer thereof; or a salt of said compound or tautomer where salt-forming groups are present;
for the manufacture of a pharmaceutical preparation useful in the treatment of a matrix metalloprotease associated disease or as antibiotics.

The more general terms used hereinabove and hereinbelow preferably have the following meanings, if not indicated otherwise:

The prefix or other word component "lower" denotes a moiety having up to and including a maximum of 7, especially up to and including a maximum of 4 carbon atoms, the moieties in question being either linear or branched with single or multiple branching if possible in view of the number of carbon atoms.

"Substituted", whereever used for a moiety, means that one or more hydrogen atoms in the respective molecule, especially up to 5, more especially up to three, of the hydrogen atoms are replaced by the corresponding number of substituents which preferably are independently selected from the group consisting of lower alkyl, for example methyl, ethyl or propyl, fluoro-lower alkyl, for example trifluoromethyl, C₆-C₁₆-aryl, especially phenyl or naphthyl (where C₆-C₁₆-aryl, especially phenyl or napthyl, is unsubstituted or substituted by one or more, especially up to three moieties selected from halogen, carboxy, lower alkoxycarbonyl, hydroxy, lower alkoxy, phenyl-lower alkoxy, lower alkanoyloxy, lower alkanoyl, amino, N-lower alkylamino, N,N-di-lower alkylamino, N-phenyl-lower alkylamino, N,N-bis(phenyl-lower alkyl)-amino, lower alkanoylamino, fluoro-lower alkyl, e.g. trifluoromethyl, and sulfo), C₃-C₁₀-cycloalkyl, hydroxy, lower alkoxy, for example methoxy, phenyl-lower alkoxy, lower alkanoyloxy, mercapto, lower alkylmercapto, phenyl-lower alkylmercapto, amino, N-lower alkylamino, N,N-di-lower alkylamino, N-phenyl-lower alkylamino, N,N-bis(phenyl-lower alkyl)-amino, lower alkanoylamino, carbamoyl-lower alkoxy, N-lower alkylcarbamoyl-lower alkoxy or N,N-di-lower alkylcarbamoyl-lower alkoxy, -NR₅R₆ wherein R₅ and R₆ independently of each other are hydrogen, C₁-C₂₀-alkyl; C₆-C₁₄-aryl which is unsubstituted or substituted with one or more substituents selected from the group consisting of lower alkyl, hydroxy-lower alkyl, hydroxy, lower alkoxy, lower alkanoyloxy, amino, mono- or di-lower alkylamino, lower alkanoylamino, carboxy, lower-alkoxycarbonyl, C₆-C₁₄-aryl-lower alkoxycarbonyl, C₆-C₁₄-aryl-oxycarbonyl, cyano, mercapto, lower alkylmercapto, sulfo, sulfamoyl, amidino, guanidino, tri-lower alkylamino, di-lower alkylamino, N-lower alkyl-N-C₆-C₁₄-aryl-lower alkyl-amino, di-C₆-C₁₄-aryl-lower alkylamino, alkanoylamino, nitro, indolyl, imidazolyl or C₆-C₁₄-aryl that is itself unsubstituted or substituted by hydroxy or lower alkoxy; or C₆-C₁₄-aryl-lower alkyl wherein aryl is unsubstituted or substituted with one or more substituents selected from the group consisting of lower alkyl, hydroxy-lower alkyl, hydroxy, lower alkoxy, lower alkanoyloxy, amino, mono- or di-lower alkylamino, lower alkanoylamino, carboxy, lower-alkoxycarbonyl, C₆-C₁₄-aryl-lower alkoxycarbonyl, C₆-C₁₄-aryl-oxycarbonyl, cyano, mercapto, lower alkylmercapto, sulfo, sulfamoyl, amidino, guanidino, tri-lower alkylamino, di-lower alkylamino, N-lower alkyl-N-C₆-C₁₄-aryl-lower alkyl-amino, di-C₆-C₁₄-aryl-lower alkylamino, alkanoylamino, nitro, indolyl, imidazolyl or C₆-C₁₄-aryl that is itself unsubstituted or substituted by hydroxy or lower alkoxy; lower alkanoylamino, lower alkoxycarbonyl-amino, such as tert-butyoxycarbonylamino; the radical of an amino acid, peptoid or peptide bound via the N-terminal nitrogen, where in each case functional groups are, independently of each other, present in free or protected form; the radical of an amino alcohol bound via the O of a hydroxy or the amino nitrogen where functional group are, independently of each other, present in free of protected form; carboxy, lower alkoxycarbonyl, such as tert-butoxycarbonyl, phenyl-, naphthyl- or fluorenyl-lower alkoxycarbonyl, such as benzyloxycarbonyl, lower alkanoyl, sulfo, lower alkanesulfonyl, for example methanesulfonyl (CH₃-S(O)₂-), phosphono (-P(=O)(OH)₂), hydroxy-lower alkoxyphosphoryl or di-lower alkoxyphosphoryl, carbamoyl, mono- or di-lower alkylcarbamoyl, sulfamoyl, mono- or di-lower alkylaminosulfonyl, tri-lower alkylsilyl, especially trimethylsilyl, . It goes without saying that substitutents are only at positions where they are chemically possible (= do not lead to unstable compounds), the person skilled in the art being able to decide (either experimentally or theoretically) without inappropriate effort which substitutions are possible and which are not.

Alkyl preferably has up to 24 carbon atoms, is preferably C₁-C₂₀-alkyl, more preferably lower alkyl.

Cycloalkyl preferably has up to 10 ring atoms and is more preferably C₃-C₈-cycloalkyl

Aryl preferably has up to 24 ring carbon atoms and is mono- or oligocyclic; more preferably it is mono-, bi- or tricyclic C₆-C₁₄-aryl, most preferably fluorenyl or especially phenyl or napthyl.

Heteroaryl is preferably a heterocyclic moiety unsaturated in the bonding ring and is preferably monocyclic or in a broader sense bicyclic or tricyclic; wherein at least in the ring bonding to the complementary rest of the molecule of formula I one or more, preferably one to four, especially one or two carbon atoms of a corresponding aryl moiety are substituted by a heteroatom selected from the group consisting of nitrogen, oxygen and sulfur, the bonding ring preferably having 4 to 12, especially 5 to 7 ring atoms; or is a saturated analogue of such an unsaturated heteroaryl. Preferred are imidazolyl, benzofuranyl, pyrrolyl, pyridyl, pyrimidinyl, indolyl, quinolyl or saturated analogs thereof.

Halogen is preferably fluoro, chloro, bromo or iodo, especially fluoro or chloro.

Substituted amino is especially -NR₅R₆ as defined in the paragraph defining "substituted".

Aminoacyl is preferably the rest of an amino acid bound via a carbonyl group; amino acids are preferably selected from the 20 natural α-amino acids glycine, alanine, valine, leucine, isoleucine, serine, threonine, methionine, cysteine, phenylalanine, tyrosine, tryptophane, asparagine, aspartic acid, glutamine, glutamic acid, histidine, arginine, lysine and proline, or of an natural or unnatural amino acid, especially selected from 3-aminopropionic acid, 2-, 3- or 4-aminobutyric acid, 3-, 4- or 5-aminopentanoic acid, 3-, 4-, 5- or 6-aminohexanoic acid, norvaline, homoserine, 4-aminophenylalanine, 4-chlorophenylalanine, 4-carboxyphenylalanine, β-phenylserine, phenylglycine, α-naphthylalanine, cyclohexylalanine, cyclohexylglycine, aspartic acid-β-phenyl-lower-alkylester, aminomalonic acid, aminomalonic acid-monoamide, glutamic acid-γ-phenyl-lower alkylester, δ-hydroxylysine, ornithine, α,γ-diaminobutyric acid and α,β-diaminopropionic acid. In each of these rests, functional groups may be free or in protected form, e.g. carboxy as lower-alkoxycarbonyl, amino as lower-alkoxycarbonylamino.

Substituted hydroxy is preferably esterified or etherified hydroxy, especially lower alkoxy, phenyl-lower alkoxy or phenoxy.

Alkenyl preferably has up to 24 carbon atoms; preferred is C₂-C₂₀alkenyl with one or more double bonds, especially C₃-C₇-alkenyl.

Alkinyl preferably has up to 24 carbon atoms; preferred is C₂-C₂₀alkinyl with one or more double bonds, especially C₃-C₇-alkinyl.

Substituted mercapto is preferably esterified or etherified mercapto, especially lower alkoxy, phenyl-lower alkoxy or phenoxy.

The radical of an amino acid bound via the N-terminal nitrogen or, if present, a non-terminal amino or hydroxy, where in each case functional groups are, independently of each other, present in free or protected form, is especially the corresponding radical of an amino acid as mentioned under "aminoacyl", most especially alanine, valine, leucine or isoleucine.

A peptoid radical preferably is a radical which is built up from 2 or more glycine radicals, if more than one, then connected by peptide bonds, where each glycine radical at the nitrogen, independently of the other, carries an unsubstituted or substituted alkyl, unsubstituted or substituted aryl or unsubstituted or substituted heteroaryl moiety, preferably a radical corresponding to the side chains of the amino acids other than glycine mentioned under "aminoacyl". The peptoid radical may preferably bound via the C-terminal carboxy or the N-terminal amino of a glycyl moiety, but also via the oxygen of a hydroxy or the sulfur of a mercapto group or an amino or carboxy group if such are present in the side chain bound to a glycine N-atom.

The peptide may be one composed of two or more, preferably up to five, amino acids, preferably those mentioned under "aminoacyl", which are bound to each other via peptide bonds, and may be bound via a C-terminal carboxy group or an N-terminal amino group or any appropriate group present in the side chains, e.g. a mercapto derived S, a hydroxy derived O, a carboxy derived -C(=O)- or an amino derived -NH-.

A radical of an amino alcohol bound via the O of a hydroxy or the amino nitrogen, where functional groups are, independently of each other, present in free or protected form, is a radical of an amino acid where the carboxy group is replaced with a hydroxymethyl group, preferably the radical of an aminoalcohol corresponding to one of the amino acids mentioned under "aminoacyl" which is bound via the hydroxy of the hydroxymethyl group just mentioned or via its amino group.

In a bridge in the form of a bivalent organic moiety formed from R₂ and R₃ together, preferably the bivalent moiety has the subformula (A), 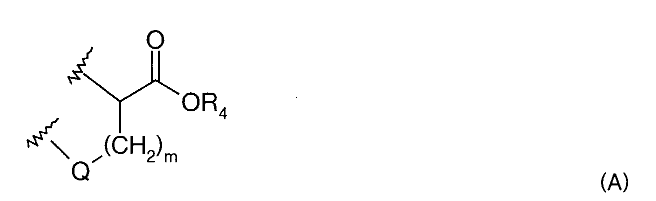 wherein m is 1 to 5 and Q is oxygen or sulfur, and which is bound at the site of R₂ via the carbon (upper bond in subformula A) and at the site of R₃ via the Q (lower bond in subformula A).

An aromatic C₆-C₁₄- or an aliphatic C₂-C₁₄-dicarbonic acid imide bonded via its imide nitrogen is preferably the corresponding moiety where the two binding carbonyl groups are bound to vicinal carbon atoms, especially succinimido or phthalimido.

Salts are especially the pharmaceutically acceptable salts of compounds of formula I (or a tautomer or mixture of tautomers thereof).

Such salts are formed, for example, as acid addition salts, preferably with organic or inorganic acids, from compounds of formula I (or a tautomer or mixture of tautomers thereof) with a basic nitrogen atom, especially the pharmaceutically acceptable salts. Suitable inorganic acids are, for example, halogen acids, such as hydrochloric acid; sulfuric acid; or phosphoric acid. Suitable organic acids are, for example, carboxylic, phosphonic, sulfonic or sulfamic acids, for example acetic acid, propionic acid, octanoic acid, decanoic acid, dodecanoic acid, glycolic acid, lactic acid, 2-hydroxybutyric acid, malic acid, benzoic acid, methane- or ethanesulfonic acid, benzenesulfonic acid, N-cyclohexylsulfamic acid, or other organic protonic acids, such as ascorbic acid. In the presence of negatively charged groups, such as carboxy or sulfo, salts may also be formed with bases, e.g. metal or ammonium salts, such as alkali metal or alkaline earth metal salts, for example sodium, potassium, magnesium or calcium salts, or ammonium salts with ammonia or suitable organic amines, such as tertiary monoamines, for example triethylamine or tri(2-hydroxyethyl)amine, or heterocyclic bases, for example N-ethyl-piperidine or N,N'-dimethylpiperazine. In the presence of both a basic and an acid group in the same molecule, a compound of formula I (or a tautomer thereof) may also form internal salts.

For isolation or purification purposes it is also possible to use pharmaceutically unacceptable salts, for example picrates or perchlorates. Only the pharmaceutically acceptable salts or free compounds (if the occasion arises, in the form of pharmaceutical preparations) attain therapeutic use, and these are therefore preferred.

In view of the close relationship between the novel compounds in free form and in the form of their salts, including those salts that can be used as intermediates, for example in the purification or identification of the novel compounds, their tautomers or tautomeric mixtures and their salts, any reference hereinbefore and hereinafter to compounds of the formula I is to be understood as referring also to the corresponding tautomers of compounds of the formula I; tautomeric mixtures of compounds of the formula I; or salts of any of these, as appropriate and expedient and if not mentioned otherwise.

Tautomers are especially possible in the case where substituent R₃ in formula I is bound via a nitrogen with at least one hydrogen bound to it, having the partial formulae 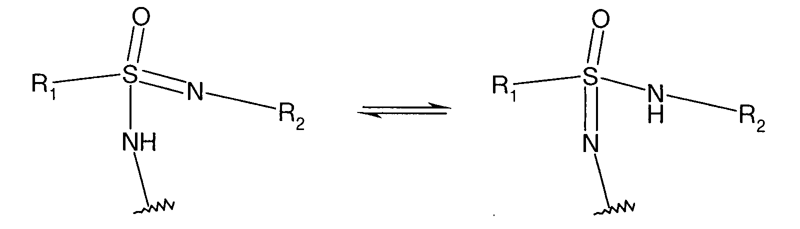 wherein R₁ and R₂ have the meanings given for compounds of the formula I. Where different tautomeric forms are possible, they may, depending on the state of the compound (e.g. in free, salt or solid form) also be present at the same time, e.g. in an equilibrium.

Any asymmetric carbon atoms or especially the central sulfur may be present in the (R)-, (S)-or (R,S)-configuration, preferably in the (R)- or (S)-configuration. Substituents at a double bond or a ring may be present in cis- (= Z-) or trans (= E-) form; especially the moiety R₂ may be present in two configurations with respect to the double bond of the nitrogen to which it is bound. The compounds may thus be present as mixtures of isomers or as pure isomers, preferably as enantiomer-pure diastereomers or enantiomers.

A matrix metalloprotease dependent disease is especially a tumor disease, including metastasis, especially lung carcinoma, but also selected from rheumatoid arthritis or other autoimmune diseases, including multiple sclerosis, Systemic Lupus erythematodes, Polymyositis, Hashimoto's thyroiditis, primary myxoedema, Grave's disease, pernicious anaemia, autoimmune atrophic gastritis, Addison's disease, ovarian failure, premature menopause, insulin-dependent diabetes mellitus, Goodpasture's syndrome, myasthenia gravis, Lambert-Eaton syndrome, pemphigus, bulous pemphigoid, psoriasis, Crohn's disease, sympathetic ophthalmia, autoimmune uveitis, multiple sclerosis, autoimmune hemolytic anaemia, autoimmune thrombocytic purpura, autoimmune neutropenia, primary biliary cirrhosis, chronic active hepatitis, cryptogenic cirrhosis, ulcerative colitis, Sjögren's syndrome, antiphospholipid syndrome, mixed connective tissue disease, vasculitis, vitiligo, glomerulonephritis, or the like.

Antibiotic means that the compounds of formula I can be used to combat infectious diseases caused by pathogenic microorganisms, especially bacteria, mycoplasms, fungi, like Candida albicans, other Candida spp. or yeasts, Entamoeba histolytica, or protozoa, like plasmodia (causative for malaria).

Bacteria and mycoplasms are preferred among the organisms against which this antibiotic effect is shown - especially Enterococcus faecium (including vancomycin resistant strains), Enterococcus faecalis (including vancomycin resistant strains), Staphylococcus aureus (including methicillin resistant strains), Staphylococcus haemolyticus, Streptococcus agalactieae, Streptococcus pneumoniae (including penicillin resistant strains), Streptococcus pyogenes; or further Neisseria gonorhoeae, Treponema pallidum, Chlamydia trachomatis, Chlamydia psittacci, Gardnerella vaginalis, Calymmatobacterium granulomatis, Helicobacter jejuni, Prevotella bivius, other Enterococcus spp., Peptococcus spp., Bacteroides spp., Mobiluncus, Enterobacteriae, such as Shigella spp., Salmonella spp., Pseudomonas aeruginosa, Mycobacterium leprae, Mycobacterium tuberculosis, Corynebacterium diphtheriae, Clostridium spp., Yersinia pestis, Vibrio cholerae or among the mycoplasms Mycoplasma hominis, Ureaplasma urealyticum, Mycoplasma genitalium. Preferred are gram-positive (aerobic) microorganisms. Preferred are microorganisms that are already resistant against one or more standard antibiotics.

A disease accessible to antibiotic treatment is especially one caused by a pathogenic microorganism mentioned in the last two paragraphs.

The inhibitory effect on metalloproteinases *in* vitro can be shown, for example, by the methods shown in Example 11 below where inhibition of collagen type 4 degradation by the following MMPs (and thus their collagenase type IV activity) is examined: MMP2, MMP3, MMP7 and MMP9. Here the compounds of formula I preferably show inhibition with an ED₅₀ (dose where half-maximal inhibition is found compared with control without inhibitor) in the range of 10⁻⁵ to 10⁻⁹ M.

The antiproliferative effect against tumors can e.g. be demonstrated *in vivo* in animal models, such as the nude mouse xenotransplant model: female BALB/c nude mice (8-12 weeks old, for example Novartis Animal Farm, Sisseln, Switzerland) are kept under sterile conditions with water and feed *ad libitum.* Tumors are induced by subcutaneous injection of tumor cells (for example the MCF-7 breast adenocarcinoma cell line, the MDA-MB 468 breast adenocarcinoma cell line , the MDA-MB 231 breast adenocarcinoma cell line, the Colo 205 colon carcinoma cell line, the HCT 116 colon carcinoma cell line, the DU145 prostate carcinoma cell line DU 145 or the PC-3 prostate carcinoma cell line PC-3; American Type Culture Collection (ATCC), Rockville, MD, USA) into carrier mice. The resulting tumors pass through at least three consecutive transplantations before the start of treatment. Tumor fragments (about 25 mg) are implanted subcutaneously in the left flank of the animals using a 13-gauge trocar needle under Forene® anaesthesia (Abbott, Switzerland). Treatment with the test compound is started as soon as the tumor has reached a mean volume of 100 mm³. Tumor growth is measured two to three times a week and 24 hours after the last treatment by determining the length of two perpendicular axes. The tumor volumes are calculated in accordance with published methods (see Evans et al., Brit. J. Cancer 45, 466-8 [1982]). The antitumor efficacy is determined as the mean increase in tumor volume of the treated animals divided by the mean increase in tumor volume of the untreated animals (controls) and, after multiplication by 100, is expressed as T/C%. Tumor regression (given in %) is reported as the smallest mean tumor volume in relation to the mean tumor volume at the start of treatment. The test compound is administered daily by gavage. In vivo tumor inhibition can e.g. be found at around 1 to 200 mg/kg and day administration of a compound of the formula I.

For antibiotic activity, the "Methods for Dilution Antimicrobial Susceptibility Tests for Bacteria that Grow Aerobically" from the US National Committee for Clinical Laboratory Standards are used (see Example 12 for details). Here, antibiotic activity can be found with compounds of the formula I in the range from 10⁻³ to 10⁻⁹ M, preferably 10⁻⁶ to 10⁻⁹ M.

For antitumor treatment, preferably the USE of compounds of the formula I given herein is meant other than those wherein R₁ is unsubstituted aryl, preferably also substituted aryl; R₂ is a moiety of the formula -N(Rₐ)(R_{b}) wherein Rₐ is alkyl, alkenyl, hydroxyalkyl, phenylalkyl, carboxyalkyl or dower alkoxyalkyl, and R_{b} is hydrogen or methyl; and R₃ is -(C=O)-N(R_{c})(R_{d}) or -C(=ORₑ)=N-R_{d} wherein R_{c} is hydrogen or lower alkyl; R_{d} is p-chloro-, p-bromo-, p-iodo- or p-lower alkyl-phenyl; and Rₑ is carboxy or lower alkoxycarbonyl.

### Preferred embodiments of the invention:

In the following preferred embodiments according to the invention, more general terms can, independently of each other, in groups or collectively, be replaced with the more specific definitions provided above, thus providing more preferred embodiments of the invention.

Where the term USE of a compound is used herein, it relates to the use of said compound in the preparation of a pharmaceutical composition for the treatment of a matrix metalloprotease (MMP) dependent disease and/or for antibiotic treatment; to the use as matrix metalloprotease inhibitor and/or antibiotic; to a method of treatment comprising said compound to an animal, especially a human, suffering from a MMP dependent disease and/or a disease accessible to antibiotic treatment; and/or to a pharmaceutical preparation comprising said compound for the treatment of a matrix metalloprotease dependent disease and/or antibiotic treatment.

The invention preferably relates to the USE of a compound of the formula I, wherein R₁ is C₁-C₂₀-alkyl; C₆-C₁₄-aryl; C₆-C₁₄-aryl substituted by one or more substituents selected from the group consisting of lower alkyl, hydroxy-lower alkyl, hydroxy, lower alkoxy, lower alkanoyloxy, amino, mono- or di-lower alkylamino, lower alkanoylamino, carboxy, lower-alkoxycarbonyl, C₆-C₁₄-aryl-lower alkoxycarbonyl, C₆-C₁₄-aryl-oxycarbonyl, cyano, mercapto, lower alkylmercapto, sulfo, sulfamoyl, amidino, guanidino, tri-lower alkylamino, di-lower alkylamino, N-lower alkyl-N-C₆-C₁₄-aryl-lower alkyl-amino, di-C₆-C₁₄-aryl-lower alkylamino, alkanoylamino, nitro, indolyl, imidazolyl and C₆-C₁₄-aryl that is itself unsubstituted or substituted by hydroxy or lower alkoxy; C₆-C₁₄-aryl-lower alkyl; or C₆-C₁₄-aryl-lower alkyl wherein aryl is substituted by one or more substituents selected from the group consisting of lower alkyl, hydroxy-lower alkyl, hydroxy, lower alkoxy, lower alkanoyloxy, amino, mono- or di-lower alkylamino, lower alkanoylamino, carboxy, lower-alkoxycarbonyl, C₆-C₁₄-aryl-lower alkoxycarbonyl, C₆-C₁₄-aryl-oxycarbonyl, cyano, mercapto, lower alkylmercapto, sulfo, sulfamoyl, amidino, guanidino, tri-lower alkylamino, di-lower alkylamino, N-lower alkyl-N-C₆-C₁₄-aryl-lower alkyl-amino, di-C₆-C₁₄-aryl-lower alkylamino, alkanoylamino, nitro, indolyl, imidazolyl or C₆-C₁₄-aryl that is itself unsubstituted or substituted by hydroxy or lower alkoxy; C₆-C₁₄-aryl-lower alkyl;
R₂ is C₁-C₂₀-alkyl; -CH(R₇)-C(=O)OR₄; -CH(R₇)-CH₂OH; C₆-C₁₄-aryl; C₆-C₁₄-aryl substituted by one or more lower alkyl moieties; C₆-C₁₄-aryl-lower alkyl; the radical of an amino acid, peptoid or a peptide bound via the N-terminal nitrogen or the C-terminal carbonyl or a non-terminal amino, carbonyl or hydroxy; or the radical of an amino alcohol bound via the O of a hydroxy or the amino nitrogen, where in each case functional groups are, independently of each other, present in free or protected form; C₆-C₁₄-aryloxy; alkoxy; C₆-C₁₄-arylthio or alkylthio;
R₃ is C₁-C₂₀-alkyl; -CH(R₇)-C(=O)OR₄; -NH-CH(R₇)C(=O)OR₄; -CH(R₇)-C(=O)-Rₓ; C₆-C₁₄-aryl; C₆-C₁₄-aryl; C₆-C₁₄-aryl-lower alkyl substituted with one or more substituents selected from the group consisting of lower alkyl, hydroxy, lower alkoxy, amino, carboxy, lower-alkoxycarbonyl, C₆-C₁₄-aryl-lower alkoxycarbonyl, C₆-C₁₄-aryl-oxycarbonyl, cyano, mercapto, lower alkylmercapto, sulfo, sulfamoyl, amidino, guanidino, tri-lower alkylamino, di-lower alkylamino, N-lower alkyl-N-C₆-C₁₄-aryl-lower alkyl-amino, di-C₆-C₁₄-aryl-lower alkylamino, alkanoylamino, nitro, indolyl, imidazolyl or C₆-C₁₄-aryl that is itself unsubstituted or substituted by hydroxy or lower alkoxy; unsubstituted or substituted C₆-C₁₄-aryloxy or -thio, if substituted, then preferably with one or more substituents selected from the group consisting of lower alkyl, hydroxy, lower alkoxy, amino, carboxy, lower-alkoxycarbonyl, C₆-C₁₄-aryl-lower alkoxycarbonyl, C₆-C₁₄-aryl-oxycarbonyl, cyano, mercapto, lower alkylmercapto, sulfo, sulfamoyl, amidino, guanidino, tri-lower alkylamino, di-lower alkylamino, N-lower alkyl-N-C₆-C₁₄-aryl-lower alkyl-amino, di-C₆-C₁₄-aryl-lower alkylamino, alkanoylamino, nitro, indolyl, imidazolyl or C₆-C₁₄-aryl that is itself unsubstituted or substituted by hydroxy or lower alkoxy; lower alkoxy; lower alkylthio; amino; the radical of an amino acid, peptoid or a peptide bound via the N-terminal nitrogen or a non-terminal amino or hydroxy, where in each case functional groups are, independently of each other, present in free or protected form; the radical of an amino alcohol bound via the O of a hydroxy or the amino nitrogen, where functional groups are, independently of each other, present in free or protected form; -NR₅R₆; -NH-C(=O)R₄; the radical of an aromatic C₆-C₁₄- or an aliphatic C₂-C₁₄-dicarbonic acid imide bonded via its imide nitrogen; hydroxy; or halogen;
with the proviso that, where amino acid, peptide, peptoid or aminoalcohol radicals are present in or as R₂ and/or R₃, they may together form a bridge between the atoms binding R₂ and R₃ in formula I, said bridge having the subformula (A), 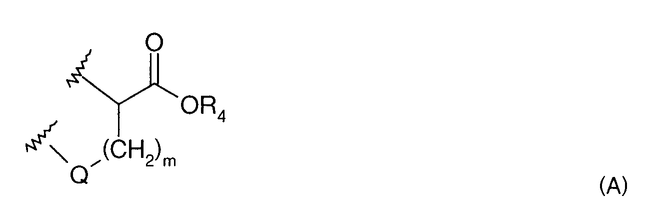 wherein m is 1 to 5 and Q is oxygen or sulfur, and which is bound at the site of R₂ via the carbon (upper bond in subformula A) and at the site of R₃ via the Q (lower bond in subformula A);
where in all cases where mentioned
R₄ is C₁-C₂₀-alkyl; C₁-C₂₀-alkyl substituted with tri-lower alkylsilyl, with lower alkoxycarbonyl or with carboxy; C₆-C₁₄-aryl; C₆-C₁₄-aryl substituted by one or more substituents selected from the group consisting of lower alkyl, hydroxy-lower alkyl, hydroxy, lower alkoxy, lower alkanoyloxy, amino, mono- or di-lower alkylamino, lower alkanoylamino, carboxy, lower-alkoxycarbonyl, C₆-C₁₄-aryl-lower alkoxycarbonyl, C₆-C₁₄-aryl-oxycarbonyl, cyano, mercapto, lower alkylmercapto, sulfo, sulfamoyl, amidino, guanidino, tri-lower alkylamino, di-lower alkylamino, N-lower alkyl-N-C₆-C₁₄-aryl-lower alkyl-amino, di-C₆-C₁₄-aryl-lower alkylamino, alkanoylamino, nitro, indolyl, imidazolyl or C₆-C₁₄-aryl that is itself unsubstituted or substituted by hydroxy or lower alkoxy; C₆-C₁₄-aryl-lower alkyl; or C₆-C₁₄-aryl-lower alkyl wherein aryl is substituted by one or more substituents selected from the group consisting of lower alkyl, hydroxy-lower alkyl, hydroxy, lower alkoxy, lower alkanoyloxy, amino, mono- or di-lower alkylamino, lower alkanoylamino, carboxy, lower-alkoxycarbonyl, C₆-C₁₄-aryl-lower alkoxycarbonyl, C₆-C₁₄-aryl-oxycarbonyl, cyano, mercapto, lower alkylmercapto, sulfo, sulfamoyl, amidino, guanidino, tri-lower alkylamino, di-lower alkyl-amino, N-lower alkyl-N-C₆-C₁₄-aryl-lower alkyl-amino, di-C₆-C₁₄-aryl-lower alkylamino, alkanoylamino, nitro, indolyl, imidazolyl or C₆-C₁₄-aryl that is itself unsubstituted or substituted by hydroxy or lower alkoxy;
Rₓ is -NR₅R₆ or is the radical of an amino acid, peptoid or a peptide bound via the N-terminal nitrogen, where in each case functional groups are, independently of each other, present in free or protected form; or the radical of an amino alcohol bound via the O of a hydroxy or the amino nitrogen where functional groups are, independently of each other, present in free or protected form;
R₅ and R₆, independently of each other, are hydrogen, C₁-C₂₀-alkyl; C₆-C₁₄-aryl which is unsubstituted or substituted with one or more substituents selected from the group consisting of lower alkyl, hydroxy-lower alkyl, hydroxy, lower alkoxy, lower alkanoyloxy, amino, mono- or di-lower alkylamino, lower alkanoylamino, carboxy, lower-alkoxycarbonyl, C₆-C₁₄-aryl-lower alkoxycarbonyl, C₆-C₁₄-aryl-oxycarbonyl, cyano, mercapto, lower alkylmercapto, sulfo, sulfamoyl, amidino, guanidino, tri-lower alkylamino, di-lower alkyl-amino, N-lower alkyl-N-C₆-C₁₄-aryl-lower alkyl-amino, di-C₆-C₁₄-aryl-lower alkylamino, alkanoylamino, nitro, indolyl, imidazolyl or C₆-C₁₄-aryl that is itself unsubstituted or substituted by hydroxy or lower alkoxy; or C₆-C₁₄-aryl-lower alkyl wherein aryl is unsubstituted or substituted with one or more substituents selected from the group consisting of lower alkyl, hydroxy-lower alkyl, hydroxy, lower alkoxy, lower alkanoyloxy, amino, mono- or di-lower alkylamino, lower alkanoylamino, carboxy, lower-alkoxycarbonyl, C₆-C₁₄-aryl-lower alkoxycarbonyl, C₆-C₁₄-aryl-oxycarbonyl, cyano, mercapto, lower alkylmercapto, sulfo, sulfamoyl, amidino, guanidino, tri-lower alkylamino, di-lower alkylamino, N-lower alkyl-N-C₆-C₁₄-aryl-lower alkyl-amino, di-C₆-C₁₄-aryl-lower alkylamino, alkanoylamino, nitro, indolyl, imidazolyl or C₆-C₁₄-aryl that is itself unsubstituted or substituted by hydroxy or lower alkoxy;
R₇ is hydrogen; unsubstituted C₁-C₂₀-alkyl; substituted C₁-C₂₀-alkyl, preferably substituted with one or more substituents selected from the group consisting of hydroxy, amino, carboxy, mercapto, lower alkylmercapto, sulfo, guanidino, tri-lower alkylamino, lower alkanoylamino, indolyl, imidazolyl or C₆-C₁₄-aryl that is itself unsubstituted or substituted by hydroxy or lower alkoxy; C₆-C₁₄-aryl; C₆-C₁₄-aryl substituted with one or more substituents selected from the group consisting of lower alkyl, hydroxy, lower alkoxy, amino, carboxy, lower-alkoxycarbonyl, C₆-C₁₄-aryl-lower alkoxycarbonyl, C₆-C₁₄-aryl-oxycarbonyl, cyano, mercapto, lower alkylmercapto, sulfo, sulfamoyl, amidino, guanidino, tri-lower alkylamino, di-lower alkylamino, N-lower alkyl-N-C₆-C₁₄-aryl-lower alkyl-amino, di-C₆-C₁₄-aryl-lower alkylamino, alkanoylamino, nitro, indolyl, imidazolyl or C₆-C₁₄-aryl that is itself unsubstituted or substituted by hydroxy or lower alkoxy; C₆-C₁₄-aryl-lower alkyl; amino; N-mono- or N,N-di-lower alkylamino; N-C₆-C₁₄-aryl-lower alkylamino; N- C₆-C₁₄-aryl-lower alkyl-N-loweralkylamino; or di-( C₆-C₁₄-aryl-lower alkyl)-amino
with the proviso that if R₁ is phenyl and R₂ is -C(=O)-CH(R₇)-NHR₅, then R₃ is other than -CH₂-C(=O)-NH-CH(R₇)-C(=O)O-R₄, alkyl and carboxy-lower alkyl;
and/or a tautomer thereof; or a salt of said compound or tautomer.

The invention more preferably relates to a novel compound of the formula I as such, especially its USE, wherein
R₁ is C₆-C₁₄-aryl substituted by lower alkyl;
R₂ is -CH(R₇)-C(=O)OR₄; or the radical of an amino acid bound via the C-terminal carbonyl
where functional groups are present in free or protected form;
R₃ is C₁-C₂₀-alkyl, -CH(R₇)C(=O)OR₄; -NH-CH(R₇)C(=O)OR₄, -CH(R₇)C(=O)-Rₓ; the radical of an aromatic C₆-C₁₄-dicarbonic acid imide bound via its imide nitrogen; C₆-C₁₄-aryloxy; or lower alkylthio;
or R₁ and R₂ together form a bridge of the formula (A) shown in claim 1 wherein
m is 1, and
Q is oxygen;
which bridge is bound at the site of R₂ via the carbon (upper bond in subformula A) and at the site of R₃ via the Q (lower bond in subformula A);
where in all cases where mentioned
R₄ is C₁-C₂₀-alkyl that is unsubstituted or substituted with tri-lower alkylsilyl or lower alkoxycarbonyl; or C₆-C₁₄-aryl-lower alkyl;
Rₓ is the radical of an amino acid bound via its amino nitrogen wherein other functional groups are present in free or in protected form; and
R₇ is hydrogen, lower alkyl or amino;
and/or a tautomer thereof; or a salt of said compound or tautomer.

The invention still more preferably relates to a novel compound of the formula I as such, especially its USE, wherein
R₁ is phenyl substituted by lower alkyl;
R₂ is -CH(R₇)-C(=O)OR₄; or the radical of the amino acid alanine, valine, leucine or isoleucine bound via the C-terminal carbonyl where the amino group is present in free or (especially lower alkoxycarbonyl) protected form;
R₃ is lower alkyl, -CH(R₇)C(=O)OR₄; -NH-CH(R₇)C(=O)OR₄, -CH(R₇)C(=O)-Rₓ; the radical of phthalic imide bound via its imide nitrogen; phenyloxy; or lower alkylthio;
or R₁ and R₂ together form a bridge of the formula (A) shown in claim 1 wherein
m is 1, and
Q is oxygen;
which bridge is bound at the site of R₂ via the carbon (upper bond in subformula A) and at the site of R₃ via the Q (lower bond in subformula A);
where in all cases where mentioned
R₄ is lower alkyl that is unsubstituted or substituted with tri-lower alkylsilyl or lower alkoxycarbonyl; or phenyl-lower alkyl;
Rₓ is the radical of an amino acid selected from the group consisting of glycine, alanine, valine, leucine and isoleucine, bound via its amino nitrogen wherein the carboxy group is present in free form or in (preferably lower alkoxy esterified) protected form; and
R₇ is hydrogen, lower alkyl or amino;
and/or a tautomer thereof; or a salt of said compound or tautomer.

The invention relates most especially to the USEs, as well as to the novel compounds as such, or their pharmaceutically acceptable salts, provided in the Examples given below.

The compounds of the formula I can be prepared according to methods that *per se* are known in the art, though they have not yet been used for the novel compounds of the invention. For example, appropriate methods can be found in EP 0 173 498 which in this respect is enclosed here for reference.

Preferably, the compounds of the invention can be prepared by
oxidising a compound of the formula II, 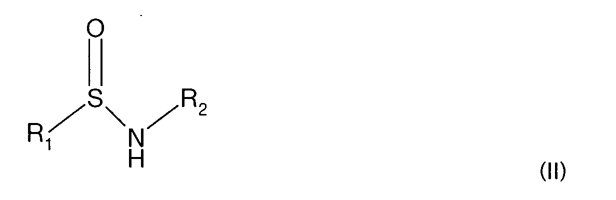 wherein R₁ and R₂ have the meanings given for a compound of the formula I, and,
a) for the synthesis of a compound of the formula I wherein R₁ is as defined under formula I, while R₂ and R₃ together are moieties other than a bridge formed by R₂ and R₃ together, treating the resulting product in parallel to the oxidation reaction or subsequently with a nucleophilic compound of the formula III

   R₃-M (III)

   wherein R₃ has the meanings given for compounds of the formula I and M is (if R₃ is bound via an O, N or S forming part of the respective moiety R₃) H or M is a metal (then the compound of formula III is an organometallic nucleophile), preferably an alkaline metal, especially potassium, sodium or lithium, or Mg-Hal or ZnHal wherein Hal is halogen, preferably chloro, bromo or iodo, or (if R₃ is bound via a carbon atom) M is halogen, especially fluoro, chloro, bromo or iodo, or
b) for the synthesis of a compound of the formula I wherein R₁ is as defined under formula I, while R₂ and R₃ together form a bridge in the form of a bivalent moiety, a moiety R₂ in formula II carrying a nucleophilic group is, in parallel to the above oxidation reaction or subsequently, cyclized with the sulfur carrying R₁ in formula II to yield the corresponding bridge formed from R₂ and R₃ together;
and, if desired, converting an obtained compound of the formula I into a different compound of the formula I, converting an obtained free compound of the formula I into a salt, converting an obtained salt of a compound of the formula I into the free compound or a different salt, and/or isolating an isomer of a compound of formula I form a mixture of isomers of a compound of the formula I;
where in all starting materials if required functional groups that shall not take part in the reaction are present in protected form, and in order to obtain the final products protecting groups are removed.

As far as novel compounds of the formula I are prepared, this process is also part of the invention.

The oxidation reaction preferably takes place in an aprotic solvent, such as a hydrocarbon, e.g. lower alkanes, benzene, toluene or xylene, an ether, or preferably a di-lower alkylether, e.g. diethyl ether, or a cyclic ether, such as tetrahydrofurane, or a halogenated hydrocarbon, such as a chlorinated lower alkane, e.g. methylene chloride or chloroform, at preferred temperatures between - 100 ° C and the reflux temperature of the respective mixture, preferably between -78 and 0 °C, e.g. at room temperature. As oxidant, a lower alkyl hypohalogenite, especially tert-butyl hypochlorite, is used. Optionally, especially if M is hydrogen bound to an adjacent O, N or S as part of R₃ in formula III, a tertiary amine is present as base, e.g. a tri-lower alkylamine, such as triethylamine, pyridine or 4-dimethylamino-pyridine.

The following reaction a), a nucleophilic replacement, preferably takes place in an appropriate solvent, especially the same solvent as used in the oxidation, at a temperature between -100 and 40 °C, e.g. between -78 and 0 °C,
(i) where M in formula III is hydrogen (R₃ bound via O, N or S) or halogen (R₃ bound via a carbon) in the presence of a tertiary amine as base, e.g. a tri-lower alkylamine, such as triethylamine, pyridine or 4-dimethylamino-pyridine, or
(ii) where M is a metal, especially as defined above, without tertiary base.

The oxidation and reaction a) may also be conducted in parallel.

The alternative reaction b) leading to cyclization of a moiety R₂ carrying a nucleophilic group, preferably a hydroxy, mercapto or amino group, e.g. where R₂ is a hydroxy-aminoacid moiety, such as Ser (bound via its amino nitrogen or its C-terminal carboxy), in protected or unprotected form, to form a ring by reaction of the nucleophilic group is preferably conducted under conditions analogous or identical to those defined for reaction a) under (i); it may also take place in parallel to the oxidation reaction.

Among the conversions that can follow, if desired, the following is of special interest:

Compounds of the formula I wherein R₁ and R₂ have the meanings indicated for formula I and wherein R₃ is methyl can be converted into the corresponding compounds wherein R₃ is carboxymethyl by first reacting with an appropriate Lithium-organic compound at temperatures from -20 to 50 °C, e.g. at 0 °C, especially Lithium-cyclohexyl-isopropylamide, in an appropriate solvent, e.g. an ether, such as tetrahydrofurane, followed by reaction with gaseous CO₂ at preferred temperatures around -78 °C (see for example Schaffner-Seebach et al., J. Org. Chem. 42, 952 (1977) and Hwang, K.-J., et al, J. Org. Chem. 51, 99 (1986), and Bolm et al., Chem. Eur. J. 7(5), 1118-28 (2001)). The resulting cyclohexylisopropylammonium salt of the caerboxymethylated product may then be used directly or after conversion into the free carboxymethyl compound for coupling reactions with an amino, mercapto or hydroxy group of amino acids, peptides or peptoids (wherein further functional groups are, if required, present in protected form) under standard conditions for (e.g. peptide) coupling, e.g. via activated esters or in the presence of coupling agents, such as dicyclohexyl carbodiimide in the absence or presence of hydroxybenzotriazole (HOBt) or related compounds, preferably in the presence of tertiary nitrogen bases, such as 4-dimethylaminopyridine, thus yielding the corresponding aminoacyl derivatives.

Generally, where peptide or amide bonds are to be synthesized in order to obtain the desired final compounds of the formula I, preferably (a) the starting compound of formula I or (b) any complementary rest to be reacted therewith, each of which carries a carboxy group in free form or as active ester or anhydride (e.g. in the form of a toluenesulfonate), is treated with appropriate coupling reagents, e.g. hydroxybenzotriazole, in an appropriate solvent, e.g. as described above for the oxidation, especially a halogenated hydrocarbon, such as methylene chloride or chloroform, at preferred temperatures from -78 to 50 °C, e.g. from -5 to 20 °C.

After the reaction (including any conversions and/or the removal of any protecting groups) is finished (control e.g. by thin layer chromatography, GC-MS or the like), the mixture is worked up according to standard procedures in order to obtain the resulting compounds of the formula I. For example, optionally after treatment with an acidifying agent, such as an ammonium halogenide, e.g. ammonium chloride, dissolved in a solvent that is not miscible with water, e.g. a di-lower alkyl ether, such as diethyl ether, or an ester, e.g. a lower alkyl alkanoate, such as ethyl acetate, the mixture is distributed between the organic and a water phase and the organic phase comprising the desired product can then be subjected to further purification steps, such as chromatography, e.g. flash chromatography.

Functional groups of the starting compounds which should not take part in the reaction may be present in unprotected form or may be protected for example by one or more of the protecting groups mentioned hereinabove. The protecting groups are then wholly or partly removed according to one of the methods described. Protecting groups are characterized in that they can be removed conveniently without destruction of the rest of the molecule and in that they are not present in the final products, if not explicitly mentioned otherwise.

If one or more other functional groups, for example carboxy, hydroxy, amino, or mercapto, are or need to be protected in a compound of formulae II or III, because they should not take part in the reaction, these are such as are usually used in the synthesis of peptide compounds, cephalosporins or penicillins, as well as nucleic acid derivatives and sugars. The protecting groups may already be present in precursors and should protect the functional groups concerned against unwanted secondary reactions, such as acylations, etherifications, esterifications, oxidations, solvolysis, and similar reactions. In certain cases, the protecting groups may, in addition to this protection, effect a selective, typically stereoselective, course of reactions. It is a characteristic of protecting groups that they lend themselves readily, i.e. without undesired secondary reactions, to removal, typically by solvolysis, reduction, photolysis or also by enzyme activity, for example under conditions analogous to physiological conditions, and that they are not present in the end-products. A person skilled in the art knows, or can easily establish, which protecting groups are suitable with the reactions mentioned hereinabove and hereinafter.

The protection of functional groups by such protecting groups, the protecting groups themselves, their introduction and their removal are described for example in standard reference works, such as J. F. W. McOmie, "Protective Groups in Organic Chemistry", Plenum Press, London and New York 1973, in T. W. Greene and P.G.M. Wuts, "Protective Groups in Organic Synthesis", Third edition, Wiley, New York 1999; in "The Peptides"; Volume 3 (editors: E. Gross and J. Meienhofer), Academic Press, London and New York 1981, in "Methoden der organischen Chemie" (*Methods of organic chemistry*), Houben Weyl, 4th edition, Volume 15/l, Georg Thieme Verlag, Stuttgart 1974, in H.-D. Jakubke and H. Jescheit, "Aminosäuren, Peptide, Proteine" (*Amino acids, peptides, proteins*), Verlag Chemie, Weinheim, Deerfield Beach, and Basel 1982, and in Jochen Lehmann, "Chemie der Kohlenhydrate: Monosaccharide und Derivate" (*Chemistry of carbohydrates: monosaccharides and derivatives*), Georg Thieme Verlag, Stuttgart 1974.

Salts of a compound of formula I (or an N-oxide, a tautomer or a mixture of tautomers thereof) with a salt-forming group may be prepared in a manner known per se. Acid addition salts may thus be obtained by treatment with an acid or with a suitable anion exchange reagent. A salt with two acid molecules (for example a dihalogenide of a compound of formula I (or an N-oxide, a tautomer or a mixture of tautomers thereof)) may also be converted into a salt with one acid molecule per compound (for example a monohalogenide); this may be done by heating to a melt, or for example by heating as a solid under a high vacuum at elevated temperature, for example from 130 to 170°C, one molecule of the acid being expelled per molecule of a compound according to the invention. Salts can usually be converted to free compounds, e.g. by treating with suitable basic agents, for example with alkali metal carbonates, hydrogencarbonates, or hydroxides, typically potassium carbonate or sodium hydroxide.

Stereoisomeric mixtures, e.g. mixtures of diastereomers, can be separated into their corresponding isomers in a manner known per se by means of suitable separation methods. Diastereomeric mixtures for example may be separated into their individual diastereomers by means of fractionated crystallization, chromatography, solvent distribution, and similar procedures. This separation may take place either at the level of one of the starting compounds or in a compound of formula I itself. Enantiomers may be separated through the formation of diastereomeric salts, for example by salt formation with an enantiomer-pure chiral acid, or by means of chromatography, for example by HPLC, using chromatographic substrates with chiral ligands.

In any of the reactions described herein, where anhydrous conditions are required, the solvents are dried (e.g. tetrahydrofurane by distillation from sodium/benzophenone, methylene chloride by distillation from from LiAlH₄) and the reactions are preferably performed under an inert gas atmosphere, e.g. under argon, using standard Schlenk techniques.

The invention relates also to those forms of the process leading to a novel compound of the formula I, a tautomer thereof, or a salt thereof, in which one starts from a compound obtainable at any stage as an intermediate and carries out the missing steps, or breaks off the process at any stage, or forms a starting material under the reaction conditions, or uses said starting material in the form of a reactive derivative or salt, or produces a compound obtainable by means of the process according to the invention and processes said compound *in situ.* In the preferred embodiment, one starts from those starting materials which lead to the compounds described hereinabove as preferred, particularly as especially preferred, primarily preferred, and/or preferred above all.

In the preferred embodiment, a compound of formula I is prepared according to or in analogy to the processes and process steps defined in the Examples.

The compounds according to the invention, including their salts, are also obtainable in the form of hydrates, or their crystals can include for example the solvent used for crystallization (present as solvates).

### Pharmaceutical Preparations:

The present invention relates also to pharmaceutical compositions that comprise a compound of formula I as active ingredient and that can be used especially in the treatment of the diseases mentioned at the beginning. Compositions for enteral administration, such as nasal, buccal, rectal or, especially, oral administration, and for parenteral administration, such as intravenous, intramuscular or subcutaneous administration, to warm-blooded animals, especially humans, are especially preferred. The compositions comprise the active ingredient (a compound of the formula I, tautomers or tautomer mixtures thereof, or a pharmaceutically acceptable salt of any of these) alone or, preferably, together with a pharmaceutically acceptable carrier. The dosage of the active ingredient depends upon the disease to be treated and upon the species, its age, weight, and individual condition, the individual pharmacokinetic data, and the mode of administration.

The invention relates also to pharmaceutical compositions for use in a method for the prophylactic or especially therapeutic management of the human or animal body, to a process for the preparation thereof (especially in the form of compositions for the treatment metalloprotease dependent diseases or diseases that are accessible to antibiotic treatment, and to a method of treatment as mentioned above, where a compound of the formula I is used.

The invention relates also to processes and to the use of compounds of formula I for the preparation of pharmaceutical preparations which comprise compounds of formula I as active component (active ingredient).

The pharmaceutical compositions comprise from approximately 1% to approximately 95% active ingredient, single-dose administration forms comprising in the preferred embodiment from approximately 20% to approximately 90% active ingredient and forms that are not of single-dose type comprising in the preferred embodiment from approximately 5% to approximately 20% active ingredient. Unit dose forms are, for example, coated and uncoated tablets, ampoules, vials, suppositories, or capsules. Examples are capsules containing from about 0.05 g to about 1.0 g of active substance.

The pharmaceutical compositions of the present invention are prepared in a manner known *per se,* for example by means of conventional mixing, granulating, coating, dissolving or lyophilizing processes.

In one aspect, preference is given to the use of solutions of the active ingredient, and also suspensions or dispersions, especially isotonic aqueous solutions, dispersions or suspensions which, for example in the case of lyophilised compositions which comprise the active ingredient on its own or together with a carrier, can be made up before use. The pharmaceutical compositions may be sterilised and/or may comprise excipients, for example preservatives, stabilisers, wetting agents and/or emulsifiers, solubilisers, salts for regulating the osmotic pressure and/ or buffers and are prepared in a manner known *per se,* for example by means of conventional dissolving and lyophilising processes.

Suspensions in oil comprise as the oil component the vegetable, synthetic, or semi-synthetic oils customary for injection purposes. Such components are in particular liquid fatty acid esters, for example ethyl oleate, isopropyl myristate, isopropyl palmitate, "Labrasol" (Gattefossé, Paris), and/or "Miglyol 812" (Hüls AG, Germany), but especially vegetable oils such as cottonseed oil, olive oil, castor oil, sesame oil, soybean oil and more especially groundnut oil.

The manufacture of preparations for parenteral use is carried out under sterile conditions, as is the filling, for example, into ampoules, and the sealing of the containers.

Pharmaceutical compositions for oral administration can be obtained, for example, by combining the active ingredient with one or more solid carriers, if need be granulating a resulting mixture, and processing the mixture or granules, if desired, to form tablets or tablet cores, if need be by the inclusion of additional excipients.

Suitable carriers are especially fillers, such as sugars, for example saccharose, mannitol or sorbitol, cellulose preparations, and/or calcium phosphates, and also binders, such as starches, for example corn, wheat, rice or potato starch, methylcellulose, and/or polyvinylpyrrolidone, and/or, if desired, disintegrators, such as the above-mentioned starches, also carboxymethyl starch, crosslinked polyvinylpyrrolidone, alginic acid or a salt thereof, such as sodium alginate. Additional excipients are especially flow conditioners and lubricants, for example silicic acid, talc, stearic acid or salts thereof and/or polyethylene glycol, or derivatives thereof.

Tablet cores may be provided with suitable, if need be enteric, coatings, using *inter alia* concentrated sugar solutions which may comprise gum arabic, talc, polyvinylpyrrolidone, polyethylene glycol and/or titanium dioxide, or coating solutions in suitable organic solvents or, for the preparation of enteric coatings, solutions of suitable cellulose preparations, such as acetylcellulose phthalate or hydroxypropylmethylcellulose phthalate. Dyes or pigments may be added to the tablets or tablet coatings, for example to indicate different doses of active ingredient.

Orally administrable pharmaceutical compositions also include hard capsules consisting of gelatin, and also soft, sealed capsules consisting of gelatin and a plasticiser, such as glycerol or sorbitol. The hard capsules may contain the active ingredient in the form of granules, for example in admixture with fillers, such as corn starch, binders, and/or glidants, and if need be stabilisers. In soft capsules, the active ingredient is preferably dissolved or suspended in suitable liquid excipients, such as fatty oils, paraffin oil or liquid polyethylene glycols or fatty acid esters of ethylene or propylene glycol, to which stabilisers and detergents may also be added.

The compounds of formula I can be administered as such or in the form of pharmaceutical compositions, prophylactically or therapeutically, preferably in an amount effective against metalloprotease-dependent diseases or diseases accessible to antibiotic treatment, to an animal, especially a warm-blooded animal, for example a human, requiring such treatment, the compounds especially being used in the form of pharmaceutical compositions. In the case of an individual having a body weight of about 70 kg the daily dose administered is from approximately 0.1 g to approximately 5 g, preferably from approximately 0.5 g to approximately 2 g, of a compound of formula I.

### Starting materials

Starting materials of the formula II and III are known or can be prepared according to methods that are known in the art, for example as described in EP 0 552 553, EP 0 173 498 or Bolm et al., Chem. Eur. J. 7(5), 1118-28 (2001).

For example, a compound of the formula II can be prepared from the corresponding sulfinic acid of the formula IV,

R₁-S(=O)OH (IV)

wherein R₁ has the meanings given under formula II, with an anorganic or organic acid halogenide, such as sulfonylchloride or especially oxalylchloride, in an appropriate solvent, e.g. as described above for the oxidation of compounds of the formula II, especially a halogenated hydrocarbon, such as methylene chloride or ethylene chloride, at preferred temperatures from -20 to 50 °C, e.g. at room temperature, yielding the corresponding halogenide of the formula V,

R₁-S(=O)Hal (V)

wherein Hal is halogen, especially chloro, bromo or iodo.

This compound is then reacted with an nucleophile of the formula VI

R₂-M* (VI)

wherein R₂ has the meanings given for compounds of the formula I and
M*, if R₂ is bound via an O, S or N as part of the moiety R₂, is hydrogen, or,
M is a metal (then the compound of formula VI is an organometallic nucleophile), preferably as defined above for M under formula II, or,
if R₂ is bound via a carbon atom M is halogen, especially fluoro, chloro, bromo or iodo.

Starting materials of the formulae V and VI are known, can be prepared according to methods that are known in the art or are commercially available.

Where required, functional groups present in the starting materials that shall not participate in the reaction are present in protected form. Purification steps etc. and, where required, protection and deprotection are possible and conducted in analogy to the methods described above for the synthesis of compounds of the formula I.

Examples: The following Examples illustrate the invention without diminishing the scope thereof.

### Example 1: Synthesis of N-(tert-Butoxycarbonylmethyl)-S-(4-methylphenyl)-S-(N-phthalimido)-sulfoximine (see Scheme 1)

The sulfinamide (2.5 mmol) of Example 1 b) is dissolved in 20 ml dichloromethane and oxidized with tert-butyl hypochlorite at room temperature. After 2 min, the resulting solution is treated with a solution of potassium phthalimide (2.75 mmol) in 15 ml dichloromethane at room temperature. After completion of the reaction(tlc control), the solution is partitioned between ethyl acetate and water, the organic phase is dried and concentrated to yield the title compound in the form of an oil which is further purified by flash chromatography on silica gel using hexane/ethyl acetate as the eluent, yielding the title compound as pale yellow oil (84 %).

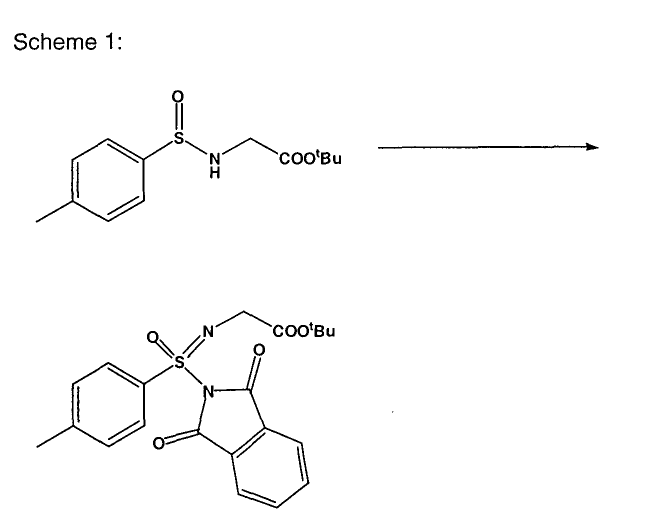

The starting materials are obtained as follows (see Scheme 2): 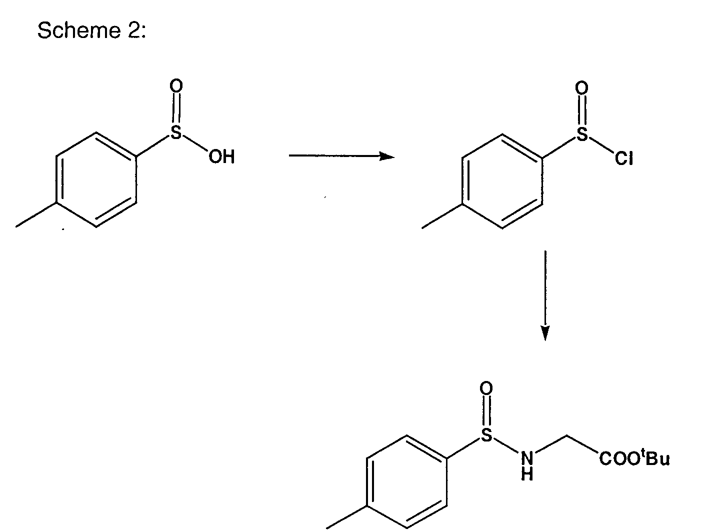
1 a) The p-toluenesulfinic acid (in the form of the sodium salt; 13 mmol) is treated with oxalyl chloride (20 mmol) in dichloromethane (25 ml) for 15 min at room temperature, yielding p-toluenesulfinyl chloride.
1 b) p-Toluenesulfinyl chloride is added to a solution of glycine tert-butylester (H-Gly-O^{t}Bu) (15 mmol) in 20 ml dichloromethane. When the reaction is complete (thin layer chromatography control), the solution is partitioned between ethyl acetate and water. The organic phase is dried and concentrated to yield N-(p-toluenesulfinyl)-glycine-tert-butylester in the form of an oil which is purified by flash chromatography on silica gel using hexane/ethylacetate as eluent, yield 92 % as a yellow oil.

The following examples are prepared in analogy to the methods described herein (in some cases specific starting materials are mentioned for more detailed illustration). Where more than one isomer is possible, the respective main (prevalent) isomer obtained is given.

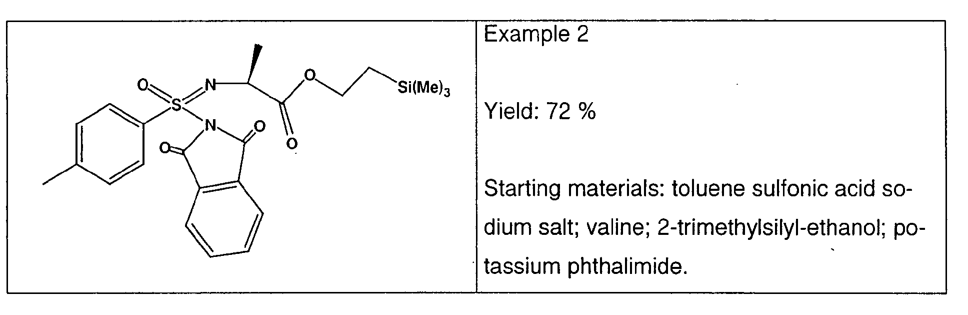

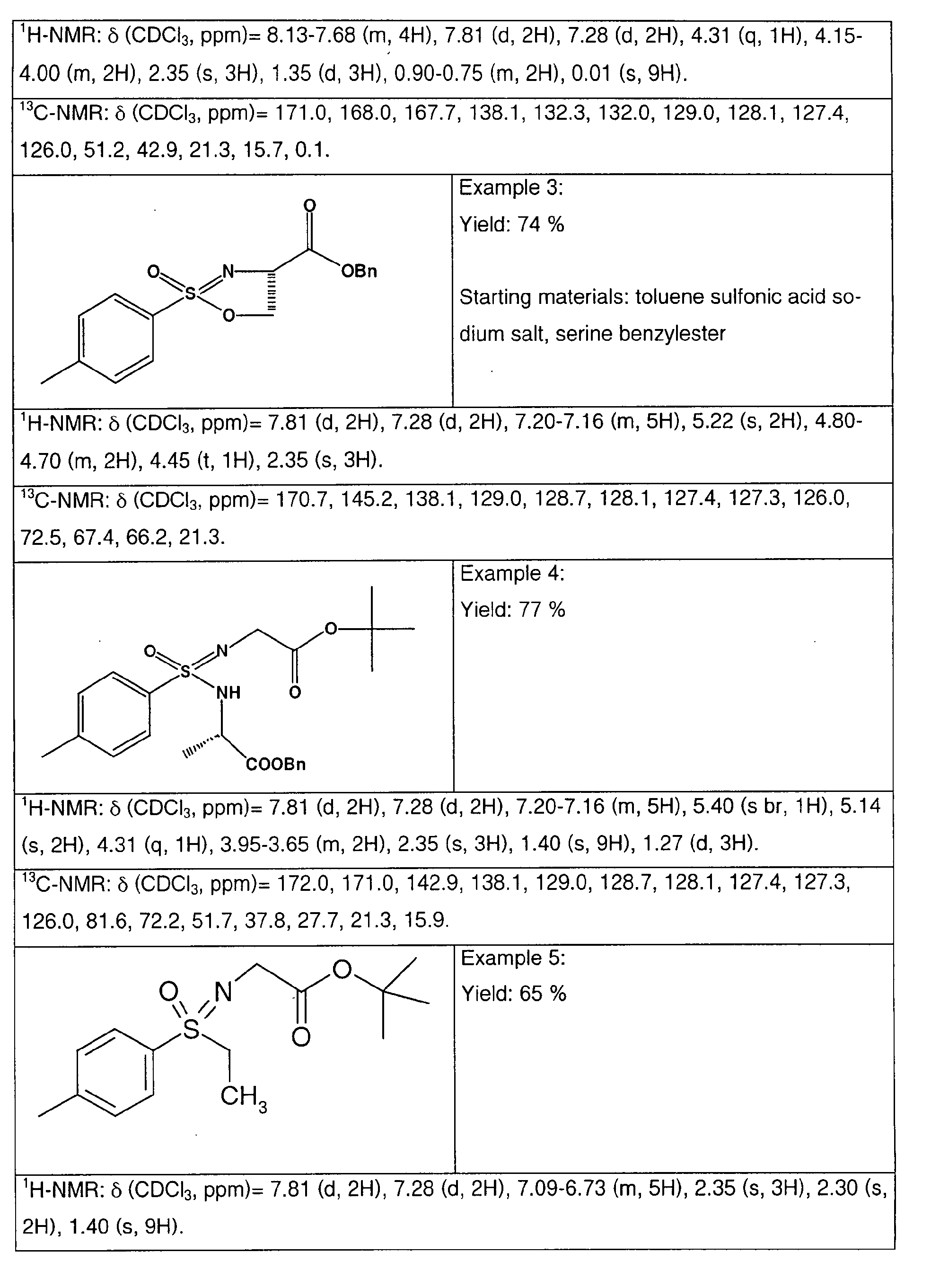

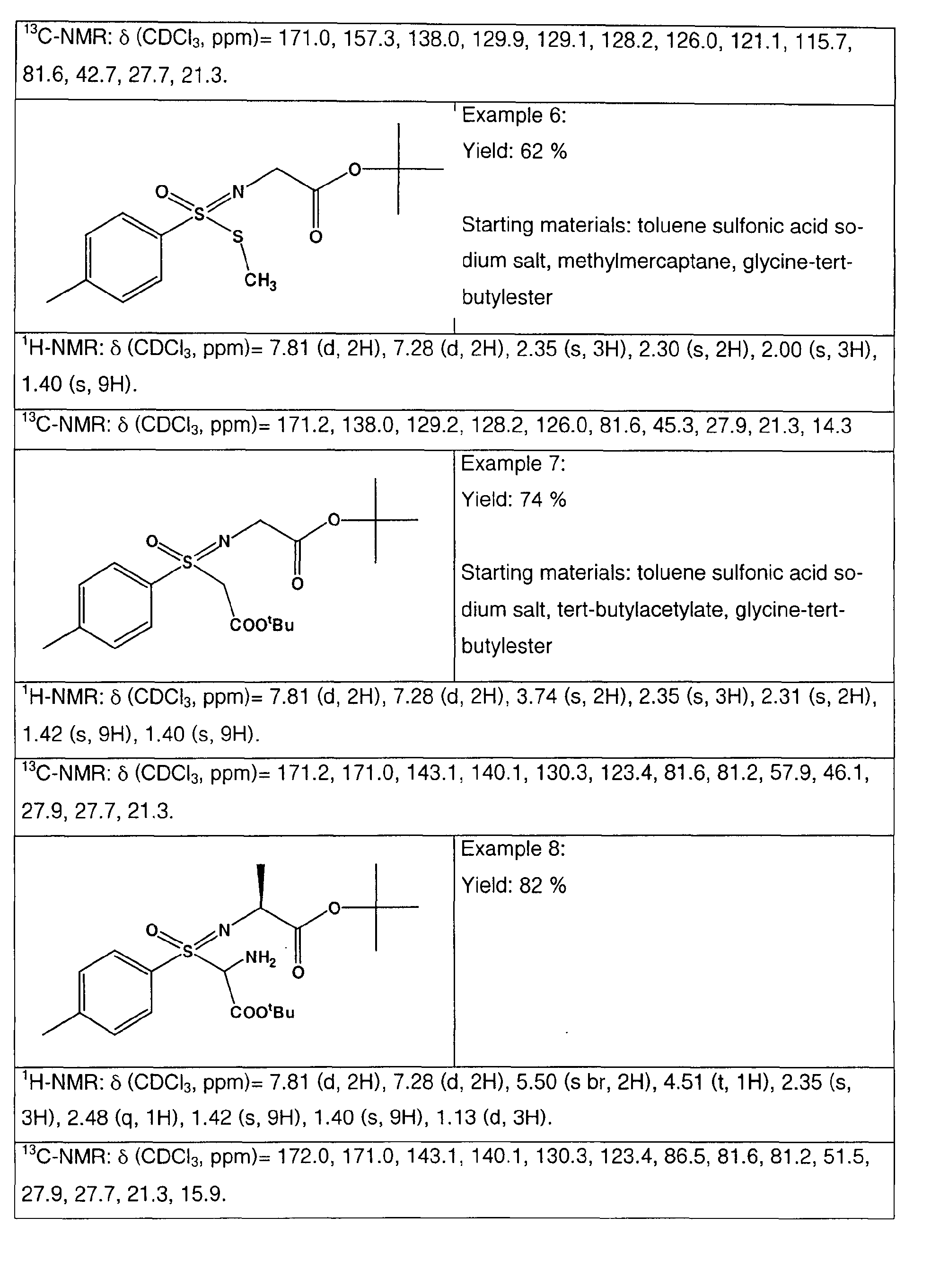

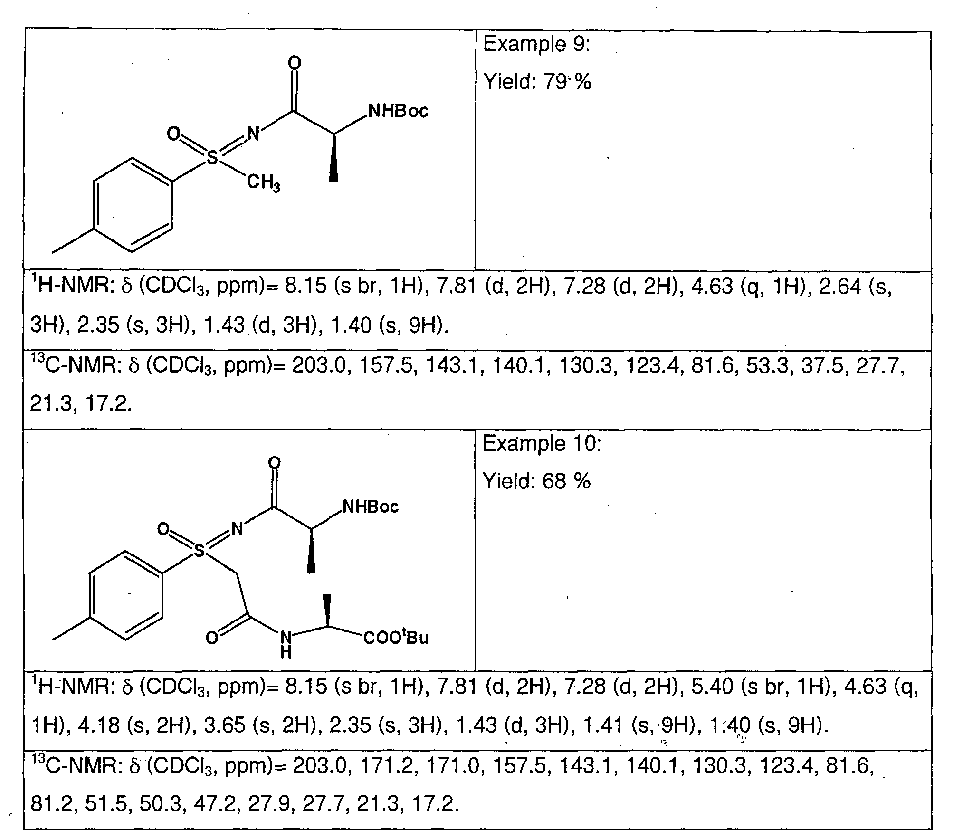

### Example 11: Inhibition of matrix metalloproteinases

### Biotests:

The inhibition of collagen type 4 degradation by the following MMPs (and thus their collagenase type IV activity) is examined: MMP2, MMP3, MMP7 and MMP9.

### Experimental:

The measurement is done in accordance with known literature procedures (see C.G. Knightet al.,, FEBS Lett. 1992, 296, 263-266; and Y. Kitamura et al., Cancer Res. 1978, 38, 4711-4716). A mixture of 30 µl of enzyme solution and 1 µl of a solution of an inhibitor according to any one of Examples 1 to 10 and 19 µl of buffer B (50mM Tris.CI/0.5 M D-Glucose/0.2 M NaCl/5mM CaCl2/0.05% Triton X-100/0.01% NaN3, pH 7,5) is incubated at room temperature for 30 min. After that period of time, 50 µl of 1 mg/ml F/TC-labelled type IV collagen solution are added, followed by incubation at 42°C for 3h. After being incubated with 300 ml of quenching solution on ice for 30 min, the mixture is centrifugated at 8000g for 10 min. Supernatant is used (340 µl) for measurement of fluorescence. Excitation and emission wavelength are 495 and 520 nm.

Inhibition is given in percent compared with control without inhibitor (= 100 %), or several concentrations of the inhibitor are examined, allowing to determin an IC₅₀ (concentration where 50 % of the collagenase type IV activity is measured compared with a control without inhibitor).

For the compounds mentioned in Examples 1 to 10, inhibition can be found in a concentration range from 10⁻⁵ to 10⁻⁹ M.

### Example 12: Antibiotic Activity of the compounds of Examples 1 to 10:

The test makes use of the "Methods for Dilution Antimicrobial Susceptibility Tests for Bacteria that Grow Aerobically", see National Committee for Clinical Laboratory Standards, 5^{th} edition, Approved Standards NCCLS Document M7-A5, Vol. 0, No. 2, NCCLS, Wayne, PA, 2000.

In brief, quantitative methods are used to determine the antimicrobial minimum inhibitory concentrations (MICs) against a variety of gram-positive (aerobic) microoorganisms, especially
Enterococcus faecium (vancomycin resistant strains)
Enterococcus faecalis (including vancomycin resistant strains)
Staphylococcus aureus (including methicillin resistant strains)
Staphylococcus haemolyticus
Streptococcus agalactieae
Streptococcus pneumoniae (penicillin resistant strains)
Streptococcus pyogenes.

These MICs provide evidence for the susceptibility of bacteria to the antimicrobial compounds. The MICs are determined using standardized procedures (dilution method).

### Example 13:

### Example 13: Formulation as solution:

The formulation comprises 100 mg of the active compound from any one of Examples 1 to 10 as active ingredient, 100 mg of racemic lactic acid (90 %), Cellulose-HP-M-603, silica gel (Aerosil 200) and deionised water (2 g).

### Example 14: Formulation as capsules:

For the preparation of 10 000 capsules comprising 100 mg of active ingredient (from any one of the afore-mentioned Examples 1 to 10) per capsule, the following constituents are processed as follows:

| | |
|---|---|
| active ingredient | 1000 g |
| ®Pluronic F 68 | 1000 g |
| hydroxypropylmethylcellulose | 1000 g |
| sesame oil | 1000 g |

(for origin of constituents see Example 10)

The sesame oil is placed in a heatable vessel (Fryma) and the ®Pluronic F 68 is scattered in. The vessel is heated at 60°C and the ®Pluronic F 68 is distributed with stirring (duration about 2 hours). With stirring and homogenisation, the mixture is cooled to about 30°C. The hydroxypropylmethylcellulose and the active ingredient are scattered in and, with stirring and homogenisation (about 1 hour), distributed in the oil mass. The suspension of pasty consistency is introduced into hard gelatin capsules (size 0; obtainable, for example, from Elanco or Parke-Davies (Caprogel)) or soft gelatin capsules (20 mm oblong; R. P. Scherer AG, Eberbach, FRG) using customary apparatus.

## Claims

1. The use of a compound of the formula I, 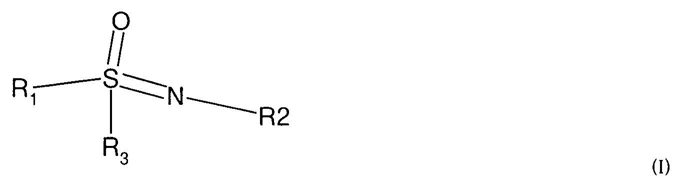 wherein R₁ is an unsubstituted or substituted moiety selected from the group consisting of alkyl, cycloalkyl, aryl and heteroaryl;
R₂ is an unsubstituted or substituted moiety selected from alkyl, cycloalkyl, aryl and heteroaryl, or is amino, halogen, substituted amino, aminoacyl or substituted hydroxy; and
R₃ is an unsubstituted or substituted moiety selected from alkyl, alkenyl, alkinyl, cycloalkyl, aryl and heteroaryl, or is substituted amino, substituted mercapto or substituted hydroxy,
or R₂ and R₃ together form a bridge in the form of a bivalent organic moiety;
or a tautomer thereof; or a salt of said compound or tautomer where salt-forming groups are present;
for the manufacture of a pharmaceutical preparation useful in the treatment of a matrix metalloprotease associated disease or as antibiotics.

2. The use according to claim 1, where a compound of the formula I is used wherein
R₁ is C₁-C₂₀-alkyl; C₆-C₁₄-aryl; C₆-C₁₄-aryl substituted by one or more substituents selected from the group consisting of lower alkyl, hydroxy-lower alkyl, hydroxy, lower alkoxy, lower alkanoyloxy, amino, mono- or di-lower alkylamino, lower alkanoylamino, carboxy, lower-alkoxycarbonyl, C₆-C₁₄-aryl-lower alkoxycarbonyl, C₆-C₁₄-aryl-oxycarbonyl, cyano, mercapto, lower alkylmercapto, sulfo, sulfamoyl, amidino, guanidino, tri-lower alkylamino, di-lower alkylamino, N-lower alkyl-N-C₆-C₁₄-aryl-lower alkyl-amino, di-C₆-C₁₄-aryl-lower alkylamino, alkanoylamino, nitro, indolyl, imidazolyl or C₆-C₁₄-aryl that is itself unsubstituted or substituted by hydroxy or lower alkoxy; C₆-C₁₄-aryl-lower alkyl; or C₆-C₁₄-aryl-lower alkyl wherein aryl is substituted by one or more substituents selected from the group consisting of lower alkyl, hydroxy-lower alkyl, hydroxy, lower alkoxy, lower alkanoyloxy, amino, mono- or di-lower alkylamino, lower alkanoylamino, carboxy, lower-alkoxycarbonyl, C₆-C₁₄-aryl-lower alkoxycarbonyl, C₆-C₁₄-aryl-oxycarbonyl, cyano, mercapto, lower alkylmercapto, sulfo, sulfamoyl, amidino, guanidino, tri-lower alkylamino, di-lower alkylamino, N-lower alkyl-N-C₆-C₁₄-aryl-lower alkyl-amino, di-C₆-C₁₄-aryl-lower alkylamino, alkanoylamino, nitro, indolyl, imidazolyl or C₆-C₁₄-aryl that is itself unsubstituted or substituted by hydroxy or lower alkoxy; C₆-C₁₄-aryl-lower alkyl;
R₂ is C₁-C₂₀-alkyl; -CH(R₇)-C(=O)OR₄; -CH(R₇)-CH₂OH; C₆-C₁₄-aryl; C₆-C₁₄-aryl substituted by one or more lower alkyl moieties; C₆-C₁₄-aryl-lower alkyl; the radical of an amino acid, peptoid or a peptide bound via the N-terminal nitrogen or the C-terminal carbonyl or a non-terminal amino, carbonyl or hydroxy; or the radical of an amino alcohol bound via the O of a hydroxy or the amino nitrogen, where in each case functional groups are, independently of each other, present in free or protected form; C₆-C₁₄-aryloxy; alkoxy; C₆-C₁₄-arylthio or alkylthio;
R₃ is C₁-C₂₀-alkyl; -CH(R₇)-C(=O)OR₄; -NH-CH(R₇)C(=O)OR₄; -CH(R₇)-C(=O)-Rₓ; C₆-C₁₄-aryl; C₆-C₁₄-aryl; C₆-C₁₄-aryl-lower alkyl substituted with one or more substituents selected from the group consisting of lower alkyl, hydroxy, lower alkoxy, amino, carboxy, lower-alkoxycarbonyl, C₆-C₁₄-aryl-lower alkoxycarbonyl, C₆-C₁₄-aryl-oxycarbonyl, cyano, mercapto, lower alkylmercapto, sulfo, sulfamoyl, amidino, guanidino, tri-lower alkylamino, di-lower alkylamino, N-lower alkyl-N-C₆-C₁₄-aryl-lower alkyl-amino, di-C₆-C₁₄-aryl-lower alkylamino, alkanoylamino, nitro, indolyl, imidazolyl or C₆-C₁₄-aryl that is itself unsubstituted or substituted by hydroxy or lower alkoxy; unsubstituted or substituted C₆-C₁₄-aryloxy or -thio, if substituted, then preferably with one or more substituents selected from the group consisting of lower alkyl, hydroxy, lower alkoxy, amino, carboxy, lower-alkoxycarbonyl, C₆-C₁₄-aryl-lower alkoxycarbonyl, C₆-C₁₄-aryl-oxycarbonyl, cyano, mercapto, lower alkylmercapto, sulfo, sulfamoyl, amidino, guanidino, tri-lower alkylamino, di-lower alkylamino, N-lower alkyl-N-C₆-C₁₄-aryl-lower alkyl-amino, di-C₆-C₁₄-aryl-lower alkylamino, alkanoylamino, nitro, indolyl, imidazolyl or C₆-C₁₄-aryl that is itself unsubstituted or substituted by hydroxy or lower alkoxy; lower alkoxy; lower alkylthio; amino; the radical of an amino acid, peptoid or a peptide bound via the N-terminal nitrogen or a non-terminal amino or hydroxy, where in each case functional groups are, independently of each other, present in free or protected form; the radical of an amino alcohol bound via the O of a hydroxy or the amino nitrogen, where functional groups are, independently of each other, present in free or protected form; -NR₅R₆; -NH-C(=O)R₄; the rest of an aromatic C₆-C₁₄- or an aliphatic C₂-C₁₄-dicarbonic acid imide bonded via its imide nitrogen; hydroxy; or halogen;
with the proviso that, where amino acid, peptide, peptoid or aminoalcohol radicals are present in or as R₂ and/or R₃, they may together form a bridge between the atoms binding R₂ and R₃ in formula I, said bridge having the subformula (A), 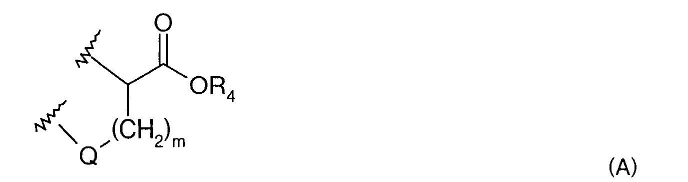 wherein m is 1 to 5 and Q is oxygen or sulfur, and which is bound at the site of R₂ via the carbon (upper bond in subformula A) and at the site of R₃ via the Q (lower bond in subformula A);
where in all cases where mentioned
R₄ is C₁-C₂₀-alkyl; C₁-C₂₀-alkyl substituted with tri-lower alkylsilyl, with lower alkoxycarbonyl or with carboxy; C₆-C₁₄-aryl; C₆-C₁₄-aryl substituted by one or more substituents selected from the group consisting of lower alkyl, hydroxy-lower alkyl, hydroxy, lower alkoxy, lower alkanoyloxy, amino, mono- or di-lower alkylamino, lower alkanoylamino, carboxy, lower-alkoxycarbonyl, C₆-C₁₄-aryl-lower alkoxycarbonyl, C₆-C₁₄-aryl-oxycarbonyl, cyano, mercapto, lower alkylmercapto, sulfo, sulfamoyl, amidino, guanidino, tri-lower alkylamino, di-lower alkylamino, N-lower alkyl-N-C₆-C₁₄-aryl-lower alkyl-amino, di-C₆-C₁₄-aryl-lower alkylamino, alkanoylamino, nitro, indolyl, imidazolyl or C₆-C₁₄-aryl that is itself unsubstituted or substituted by hydroxy or lower alkoxy; C₆-C₁₄-aryl-lower alkyl; or C₆-C₁₄-aryl-lower alkyl wherein aryl is substituted by one or more substituents selected from the group consisting of lower alkyl, hydroxy-lower alkyl, hydroxy, lower alkoxy, lower alkanoyloxy, amino, mono- or di-lower alkylamino, lower alkanoylamino, carboxy, lower-alkoxycarbonyl, C₆-C₁₄-aryl-lower alkoxycarbonyl, C₆-C₁₄-aryl-oxycarbonyl, cyano, mercapto, lower alkylmercapto, sulfo, sulfamoyl, amidino, guanidino, tri-lower alkylamino, di-lower alkylamino, N-lower alkyl-N-C₆-C₁₄-aryl-lower alkyl-amino, di-C₆-C₁₄-aryl-lower alkyl-amino, alkanoylamino, nitro, indolyl, imidazolyl or C₆-C₁₄-aryl that is itself unsubstituted or substituted by hydroxy or lower alkoxy;
Rₓ is -NR₅R₆ or is the radical of an amino acid, peptoid or a peptide bound via the N-terminal nitrogen, where in each case functional groups are, independently of each other, present in free or protected form; or the radical of an amino alcohol bound via the O of a hydroxy or the amino nitrogen where functional groups are, independently of each other, present in free or protected form;
R₅ and R₆, independently of each other, are hydrogen, C₁-C₂₀-alkyl; C₆-C₁₄-aryl which is unsubstituted or substituted with one or more substituents selected from the group consisting of lower alkyl, hydroxy-lower alkyl, hydroxy, lower alkoxy, lower alkanoyloxy, amino, mono- or di-lower alkylamino, lower alkanoylamino, carboxy, lower-alkoxycarbonyl, C₆-C₁₄-aryl-lower alkoxycarbonyl, C₆-C₁₄-aryl-oxycarbonyl, cyano, mercapto, lower alkylmercapto, sulfo, sulfamoyl, amidino, guanidino, tri-lower alkylamino, di-lower alkyl-amino, N-lower alkyl-N-C₆-C₁₄-aryl-lower alkyl-amino, di-C₆-C₁₄-aryl-lower alkylamino, alkanoylamino, nitro, indolyl, imidazolyl or C₆-C₁₄-aryl that is itself unsubstituted or substituted by hydroxy or lower alkoxy; or C₆-C₁₄-aryl-lower alkyl wherein aryl is unsubstituted or substituted with one or more substituents selected from the group consisting of lower alkyl, hydroxy-lower alkyl, hydroxy, lower alkoxy, lower alkanoyloxy, amino, mono- or di-lower alkylamino, lower alkanoylamino, carboxy, lower-alkoxycarbonyl, C₆-C₁₄-aryl-lower alkoxycarbonyl, C₆-C₁₄-aryl-oxycarbonyl, cyano, mercapto, lower alkylmercapto, sulfo, sulfamoyl, amidino, guanidino, tri-lower alkylamino, di-lower alkylamino, N-lower alkyl-N-C₆-C₁₄-aryl-lower alkyl-amino, di-C₆-C₁₄-aryl-lower alkylamino, alkanoylamino, nitro, indolyl, imidazolyl or C₆-C₁₄-aryl that is itself unsubstituted or substituted by hydroxy or lower alkoxy;
R₇ is hydrogen; unsubstituted C₁-C₂₀-alkyl; substituted C₁-C₂₀-alkyl, preferably substituted with one or more substituents selected from the group consisting of hydroxy, amino, carboxy, mercapto, lower alkylmercapto, sulfo, guanidino, tri-lower alkylamino, lower alkanoylamino, indolyl, imidazolyl or C₆-C₁₄-aryl that is itself unsubstituted or substituted by hydroxy or lower alkoxy; C₆-C₁₄-aryl; C₆-C₁₄-aryl substituted with one or more substituents selected from the group consisting of lower alkyl, hydroxy, lower alkoxy, amino, carboxy, lower-alkoxycarbonyl, C₆-C₁₄-aryl-lower alkoxycarbonyl, C₆-C₁₄-aryl-oxycarbonyl, cyano, mercapto, lower alkylmercapto, sulfo, sulfamoyl, amidino, guanidino, tri-lower alkylamino, di-lower alkylamino, N-lower alkyl-N-C₆-C₁₄-aryl-lower alkyl-amino, di-C₆-C₁₄-aryl-lower alkylamino, alkanoylamino, nitro, indolyl, imidazolyl or C₆-C₁₄-aryl that is itself unsubstituted or substituted by hydroxy or lower alkoxy; C₆-C₁₄-aryl-lower alkyl; amino; N-mono- or N,N-di-lower alkylamino; N-C₆-C₁₄-aryl-lower alkylamino; N- C₆-C₁₄-aryl-lower alkyl-N-loweralkylamino; or di-( C₆-C₁₄-aryl-lower alkyl)-amino
with the proviso that if R₁ is phenyl and R₂ is -C(=O)-CH(R₇)-NHR₅, then R₃ is other than -CH₂-C(=O)-NH-CH(R₇)-C(=O)O-R₄, alkyl and carboxy-lower alkyl; and/or a tautomer thereof; or a salt of said compound or tautomer.

3. A compound of the formula I as shown in claim 1, wherein
R₁ is C₆-C₁₄-aryl substituted by lower alkyl;
R₂ is -CH(R₇)-C(=O)OR₄; or the radical of an amino acid bound via the C-terminal carbonyl
where functional groups are present in free or protected form;
R₃ is C₁-C₂₀-alkyl, -CH(R₇)C(=O)OR₄; -NH-CH(R₇)C(=O)OR₄, -CH(R₇)C(=O)-Rₓ; the rest of an aromatic C₆-C₁₄-dicarbonic acid imide bound via its imide nitrogen; C₆-C₁₄-aryloxy; or lower alkylthio;
or R₁ and R₂ together form a bridge of the formula (A) shown in claim 1 wherein
m is 1, and
Q is oxygen;
which bridge is bound at the site of R₂ via the carbon (upper bond in subformula A) and at the site of R₃ via the Q (lower bond in subformula A);
where in all cases where mentioned
R₄ is C₁-C₂₀-alkyl that is unsubstituted or substituted with tri-lower alkylsilyl or lower alkoxycarbonyl; or C₆-C₁₄-aryl-lower alkyl;
Rₓ is the radical of an amino acid bound via its amino nitrogen wherein other functional groups are present in free or in protected form; and
R₇ is hydrogen, lower alkyl or amino;
and/or a tautomer thereof; or a salt of said compound or tautomer.

4. A compound of the formula I according to claim 3, wherein
R₁ is phenyl substituted by lower alkyl;
R₂ is -CH(R₇)-C(=O)OR₄; or the radical of the amino acid alanine, valine, leucine or isoleucine bound via the C-terminal carbonyl where the amino group is present in free or (especially lower alkoxycarbonyl) protected form;
R₃ is lower alkyl, -CH(R₇)C(=O)OR₄; -NH-CH(R₇)C(=O)OR₄, -CH(R₇)C(=O)-Rₓ; the rest of phthalic imide bound via its imide nitrogen; phenyloxy; or lower alkylthio;
or R₁ and R₂ together form a bridge of the formula (A) shown in claim 1 wherein
m is 1, and
Q is oxygen;
which bridge is bound at the site of R₂ via the carbon (upper bond in subformula A) and at the site of R₃ via the Q (lower bond in subformula A);
where in all cases where mentioned
R₄ is lower alkyl that is unsubstituted or substituted with tri-lower alkylsilyl or lower alkoxycarbonyl; or phenyl-lower alkyl;
Rₓ is the radical of an amino acid selected from the group consisting of glycine, alanine, valine, leucine and isoleucine, bound via its amino nitrogen wherein the carboxy group is present in free form or in (preferably lower alkoxy esterified) protected form; and
R₇ is hydrogen, lower alkyl or amino;
and/or a tautomer thereof; or a salt of said compound or tautomer.

5. A compound of the formula I according to claim 3, selected from N-(tert-Butoxycarbonylmethyl)-S-(4-methylphenyl)-S-(N-phthalimido)-sulfoximine or the compounds with the formulae 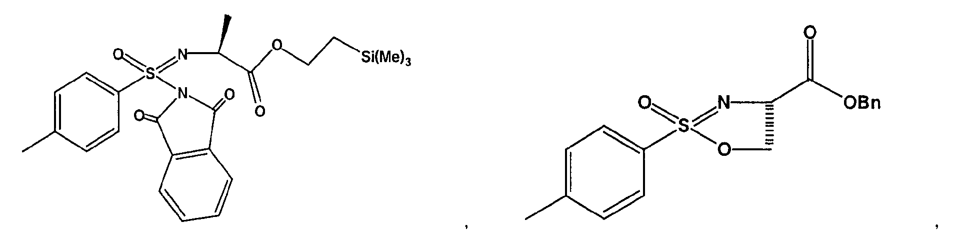 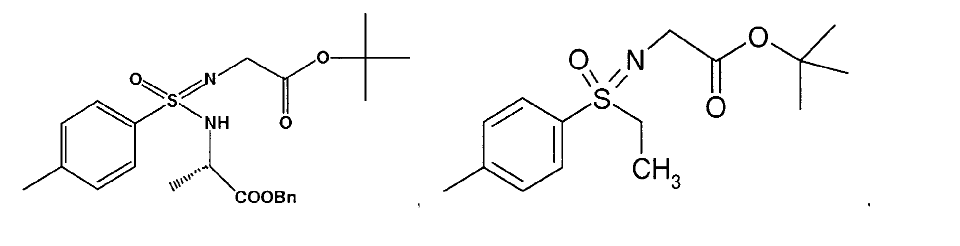 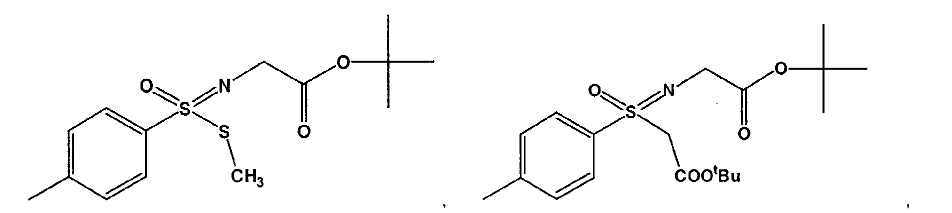 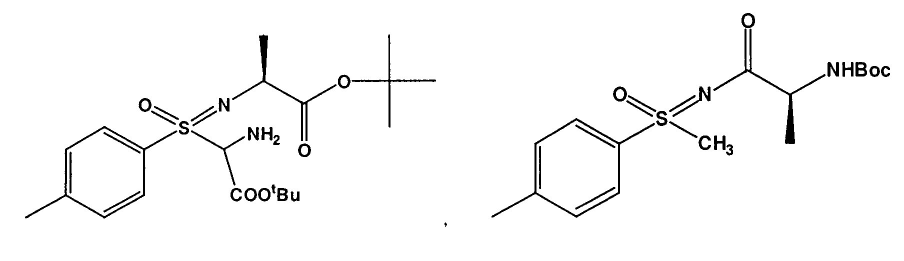 and 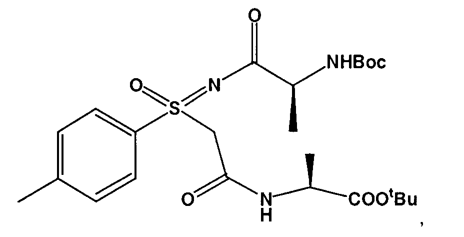 or a salt thereof.

6. A compound of the formula I, or a pharmaceutically according to any one of claims 3 to 5, or a pharmaceutically acceptable salt thereof, for use in the therapeutic treatment of an animal, especially a human.

7. The use of a compound of the formula I according to any one of claims 3 to 5 for the preparation of a pharmaceutical composition for the treatment of a metalloproteinase dependent disease or a disease accessible to antibiotic treatment.
